# EUROPEAN PATENT SPECIFICATION

(11) **EP 2 737 076 B1**
(45) Date of publication and mention of the grant of the patent: **02.03.2016**
(21) Application number: 11751557.7
(22) Date of filing: 18.07.2011
(51) Int. Cl.: C12Q 1/06

(54) **METHOD AND DEVICE FOR DETECTION AND QUANTIFICATION OF THERMODURIC MICROORGANISMS IN A PRODUCT**
VERFAHREN UND VORRICHTUNG ZUR DETEKTION UND QUANTIFIZIERUNG EINES TEMPERATURSTABILEN MIKROORGANSIMUS IN EINEM ERZEUGNIS
MÉTHODE ET DISPOSITIF POUR LA DÉTECTION ET LA QUANTIFICATION DES MICRO-ORGANISMES THERMODURIQUES DANS UN PRODUIT

(43) Date of publication of application: 04.06.2014
(73) Proprietor: Luxcel Biosciences Ltd., Cork (IE)
(72) Inventor: HYNES, James Niall, Ovens Co. Cork (IE); PAPKOVSKY, Dmitri Boris, Blarney Co. Cork (IE)
(74) Representative: Purdy, Hugh Barry
(86) International application number: PCT/EP2011/062233
(87) International publication number: WO 2013/010574

(56) References cited:
- WO-A1-2004/015413
- US-A1- 2004 241 783
- PETTIPHER G L ET AL: "RAPID ENUMERATION OF BACTERIA IN HEAT TREATED MILK AND MILK PRODUCTS USING A MEMBRANE FILTRATION EPI FLUORESCENT MICROSCOPY TECHNIQUE", 1981, JOURNAL OF APPLIED BACTERIOLOGY, VOL. 50, NR. 1, PAGE(S) 157-166, XP002662491, ISSN: 0021-8847 abstract pages 157-158
- O'MAHONY F C ET AL: "Rapid high-throughput assessment of aerobic bacteria in complex samples by fluorescence-based oxygen respirometry", February 2006 (2006-02), APPLIED AND ENVIRONMENTAL MICROBIOLOGY FEBRUARY 2006 AMERICAN SOCIETY FOR MICROBIOLOGY US, VOL. 72, NR. 2, PAGE(S) 1279 - 1287, XP002662492, page 1280 - page 1281; figure 1
- YU H ET AL: "IMMUNOMAGNETIC-ELECTROCHEMILUMINESCENT DETECTION OF ESCHERICHIA COLI O157 AND SALMONELLA TYPHIMURIUM IN FOODS AND ENVIRONMENTAL WATER SAMPLES", APPLIED AND ENVIRONMENTAL MICROBIOLOGY, AMERICAN SOCIETY FOR MICROBIOLOGY, US, vol. 62, no. 2, 1 February 1996 (1996-02-01), pages 587-592, XP000922706, ISSN: 0099-2240

## Description

### BACKGROUND

A number of food products cannot be sterilized without adversely affecting the quality and/or taste of the product. Such foods, such as milk, are often pasteurized in order to reduce the number of viable pathogens and slow microbial growth without adversely affecting the taste and quality of the product.

There are various pasteurization techniques in use today. These include Ultra High Temperature (UHT), High Temperature/Short Time (HTST), Vat or Batch (LTLT), and Extended Shelf Life (ESL). The UHT pasteurization technique heats the product to 135 °C (275 °F) for a minimum of one second. The HTST pasteurization technique heats the product to 72 °C (161 °F) for 15 to 20 seconds. The Vat or Batch pasteurization technique heats the product to 63 °C (146 °F) for 30 minutes. The ESL pasteurization technique uses even lower temperatures but in combination with microbial filtration.

While pasteurization is effective for enhancing shelf-life and reducing microbial risks, some bacteria survive pasteurization. Such bacteria are known as *thermoduric bacteria* and are most commonly associated with some contamination source. The standard test for detecting and enumerating *thermoduric bacteria* is the Laboratory Pasteurisation Count (LPC), which serves as an indicator of the effectiveness of farm sanitation and hygiene procedures. Under laboratory conditions pasteurization at lower temperatures is usually easier to implement. However, the use of lower pasteurization temperatures can be associated with a higher risk of residual contamination and hence can require more strict control measures.

LPC typically involves heating a rack of approximately 5 ml samples, each retained within in a sampling bottle, to 63°C in a water bath for 30 min, followed by immediate cooling. An aliquot of each individual temperature treated sample is then withdrawn from the bottle and deposited into an agar plate, followed by prolonged incubation (typically 24 to 72 hrs) and subsequent colony counting.

While generally effective for detecting and enumerating *thermoduric bacteria,* LPC is relatively slow and labor-intensive as it includes numerous steps involving manipulation of the samples, and results in a subjective readout.

Accordingly, a need exists for an improved method of detecting and enumerating *thermoduric bacteria.*

### SUMMARY OF THE INVENTION

The invention is directed to the detection of *thermoduric microorganisms* in a product with minimal manipulation of the sample. A first embodiment of the invention is a method of detecting the presence of *thermoduric microorganisms* in a product. The method includes the steps of (i) placing an aliquot of the product into a vessel equipped with an optical probe sensitive to a thermoduric microorganism metabolite, (ii) pasteurizing the aliquot within the vessel, (iii) incubating the pasteurized aliquot within the vessel for an incubation period, and (iv) periodically interrogating the probe during the incubation period. The interrogations measure changes in the probe reflective of changes in concentration of a thermoduric microorganism metabolite within the aliquot, thereby indicating the presence of viable *thermoduric microorganisms* in the aliquot.

*Thermoduric microorganisms* in the product prior to incubation can be enumerated by converting measured changes in the probe to a concentration of *thermoduric microorganisms* in the pasteurized aliquot based upon a known conversion algorithm.

Interrogation of the probe is preferably effected remotely, through the walls of the vessel, in order to eliminate the need to physically contact the aliquot during the testing period.

A second embodiment of the invention is a method for comparatively detecting the presence of *thermoduric microorganisms* and total microorganisms in a product. The method includes the steps of (i) obtaining a sample of the product, (ii) placing a first aliquot of the sample into a first retention chamber equipped with a first probe sensitive to a thermoduric microorganism metabolite, (iii) placing a second aliquot of the sample into a second retention chamber equipped with a second probe sensitive to a target-analyte, (d) pasteurizing the first aliquot within the first retention chamber but not the second aliquot, (e) incubating the pasteurized first aliquot within the first retention chamber and the second aliquot within the second retention chamber for an incubation period, and (f) periodically interrogating both probes during the incubation period, wherein the interrogations measure changes in the probe reflective of changes in concentration of a thermoduric microorganism metabolite within the first aliquot and changes in concentration of a target-analyte within the second aliquot, with such changes in concentration indicative of the presence of viable thermoduric microorganisms in the first aliquot and the presence of total viable microorganisms in the second aliquot.

*Thermoduric microorganisms* and total microorganisms in the product prior to incubation can be enumerated by converting measured changes in the first probe to a concentration of *thermoduric microorganisms* in the pasteurized first aliquot based upon a known conversion algorithm, and converting measured changes in the second probe to a concentration of total microorganisms in the second aliquot prior to incubation based upon a known conversion algorithm.

Interrogation of the probe is preferably effected remotely, through the walls of the vessel, in order to eliminate the need to physically contact the aliquot during the testing period.

### BRIEF DESCRIPTION OF THE DRAWINGS

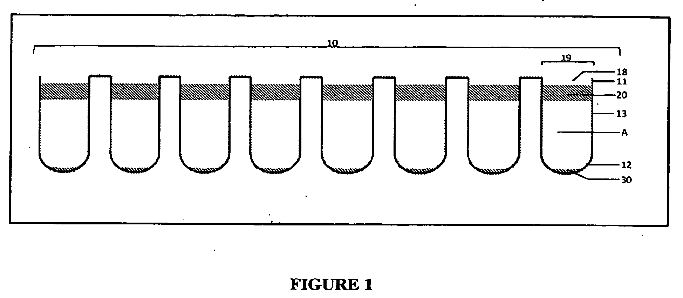

Figure 1 is a side view of one embodiment of a tool in accordance with this invention.

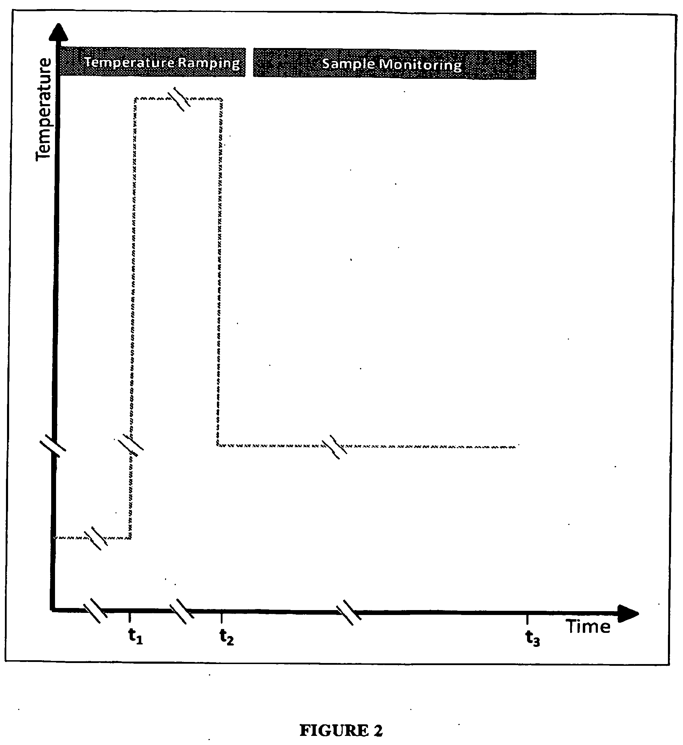

Figure 2 is a generic graph of temperature over time for an exemplary process for detecting *thermoduric microorganisms* in a product in accordance with this invention. Phase 1 (t₀ to t₁) indicates sample preparation steps (s) (no temperature control and no probe interrogation). Phase 2 (t₁ to t₂) indicates pasteurisation step (temperature ramp for defined time period without probe interrogation). Phase 3 (t₂ to t₃) indicates incubation period (incubation temperature with periodic probe interrogation).

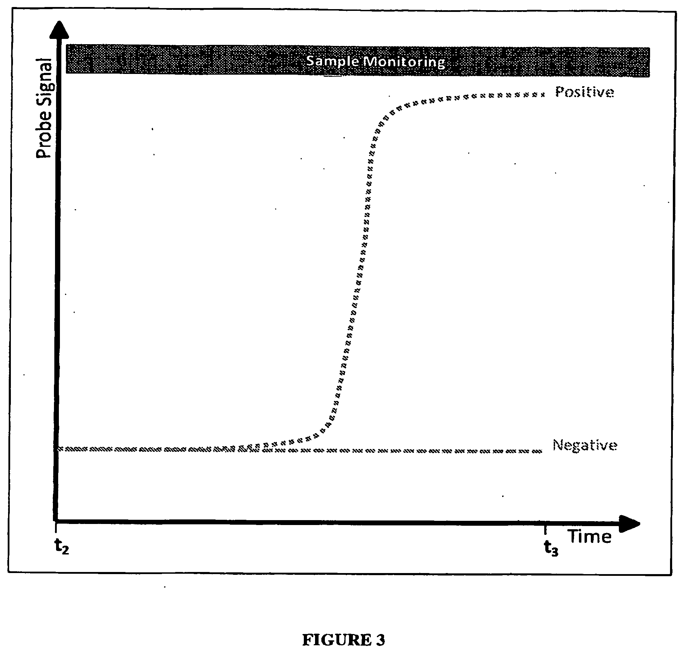

Figure 3 is a generic graph of probe signal over time for an exemplary detection of *thermoduric microorganisms* during Phase 3 (t₂ to t₃) in a product in accordance with this invention. Exemplary signal profiles for both a positive aliquot (*i.e*., an aliquot with sufficient *thermoduric microorganisms* to grow after pasteurization and consume substantially all target-analyte within the aliquot during the incubation period (Phase 3 (t₂ to t₃))), and a negative aliquot (*i.e*., an aliquot with insufficient *thermoduric microorganisms* to grow after pasteurization and consume substantially all target-analyte within the aliquot during the incubation period (Phase 3 (t₂ to t₃))).

### DETAILED DESCRIPTION OF A PREFERRED EMBODIMENT

### Definitions

As used herein, including the claims, the term "**p*asteurization***" means heating a product, typically a liquid food product susceptible to degradation at sterilization temperatures, to a specific elevated temperature below that required for sterilization for a defined length of time to slow microbial growth.

As used herein, including the claims, the phrase "***Vat Pasteurization***" (VatP) or "***Batch Pasteurization***" (LTLT) means heated to and held at 63°C (145°F) for 30 minutes.

As used herein, including the claims, the phrase "***High Temperature Short Time Pasteurization***" (HTST) means heated to and held at 72°C (162°F) for 15 seconds.

As used herein, including the claims, the phrase "***Ultra Pasteurization***" (UP) means heated to and held at 138°C (280°F) for 2 seconds.

As used herein, including the claims, the phrase "***thermoduric microorganism metabolite***" means a molecule consumed or produced during *thermoduric microorganism* metabolism.

As utilized herein, including the claims, the phrase **"*surface-to-volume ratio*"** means the amount of surface per unit volume.

As utilized herein, including the claims, the phrase **"*elevated surface-to-volume ratio***" means a surface-to-volume ratio of greater than 12:1.

As used herein, including the claims, the term "***target-analyte***" refers to a chemical substance, typically O₂, CO₂ or H⁺, constituting a *thermoduric microorganism* metabolite and capable of modulating the optical signal emanating from an optically-active material such as a photoluminescentprobe. The modulating effect may be achieved by quenching, enhancement, (de)protonation or other means.

### Nomenclature

- **10**: Vessel
- **11**: Top of Vessel
- **12**: Bottom of Vessel
- **13**: Diathermal Sidewalls of Vessel
- **18**: Opening into Retention Chamber
- **19**: Retention Chamber
- **19₁**: First Retention Chamber
- **19₂**: Second Retention Chamber
- **20**: Cap
- **30**: Probe
- **30₁**: First Probe
- **30₂**: Second Probe
- **A**: Aliquot of Product
- **A₁**: First Aliquot of Product
- **A₂**: Second Aliquot of Product

### Description

### Tool

The invention employs a tool specially adapted for use in detecting the presence of microorganisms, particularly *thermoduric microorganisms,* in a product. The tool is a small vessel **10** defining a retention chamber **19** and equipped with a probe **30** sensitive to a target-analyte in operable communication with the retention chamber **19**. The top **11** of the vessel **10** is open for allowing access to the retention chamber **19**. A cap **20** or other sealing device may be provided for sealing the top **11** of the retention chamber **19**.

Suitable vessels **10** include vials, cuvettes, multi-well plates (*e.g.,* 6, 12, 24, 48, 96 and 384 well plates), and the like.

The vessel **10** is constructed, configured and arranged to withstand pasteurization temperatures and temperature profiles, and permit any contents placed within the retention chamber **19** of the vessel **10** to be quickly heated and cooled through the diathermal sidewall(s) **13** of the vessel **10**. The vessel **10** is preferably constructed, configured and arranged such that thermal equilibration of an aliquot **A** placed into the retention chamber **19** of the vessel **10** can be achieved within ½ of the pasteurization time period, preferably within ¼^{th} of the pasteurization time period and most preferably even quicker than this.

The retention chamber **19** is preferably sized and configured to facilitate quick heating and cooling of any contents by providing an elevated surface-to-volume ratio, most preferably a high surface-to-volume ratio.

The probe **30** can be any device capable of sensing and reporting changes in a target-analyte concentration within an enclosed volume. In a preferred embodiment, the probe **30** is an optically-active, target-analyte sensitive material configured and arranged to experience changes in target-analyte concentration or partial pressure P_{A} in an aliquot **A** placed within the retention chamber **19** of the vessel **10**. The analyte-sensitive material is preferably a photoluminescent dye embedded within an analyte permeable polymer matrix. Since the preferred type of probe **30** is an optically-active, target-analyte sensitive material, and the most frequent target-analyte of interest is oxygen, the balance of the disclosure shall be based upon a photoluminescent oxygen quenched probe **30** without intending to be limited thereby.

The oxygen-sensitive photoluminescent dye may be selected from any of the well-known oxygen sensitive photoluminescent dyes. One of routine skill in the art is capable of selecting a suitable dye based upon the intended use of the probe **30**. A nonexhaustive list of suitable oxygen sensitive photoluminescent dyes includes specifically, but not exclusively, ruthenium(II)-bipyridyl and ruthenium(II)-diphenylphenanothroline complexes, porphyrin-ketones such as platinum(II)-octaethylporphine-ketone, platinum(II)-porphyrin such as platinum(II)-tetrakis(pentafluorophenyl)porphine, palladium(II)-porphyrin such as palladium(II)-tetrakis(pentafluorophenyl)porphine, phosphorescent metallocomplexes of tetrabenzoporphyrins, chlorins, azaporphyrins, and long-decay luminescent complexes of iridium(III) or osmium(II).

Typically, the hydrophobic oxygen-sensitive photoluminescent dye is compounded with a suitable oxygen-permeable and hydrophobic carrier matrix. The carrier matrix, indeed the entire probe **30**, must be able to withstand the pasteurisation and incubation conditions. Again, one of routine skill in the art is capable of selecting a suitable oxygen-permeable hydrophobic carrier matrix based upon the intended use of the probe **30** and the selected dye. A nonexhaustive list of suitable polymers for use as the oxygen-permeable hydrophobic carrier matrix includes specifically, but not exclusively, polystryrene, polycarbonate, polysulfone, polyvinyl chloride and some co-polymers.

When the probe **30** is based on the quenching of photoluminescence by an analyte, the vessel **10**, or at least that portion of the vessel **10** coated with the probe **30**, must allow radiation at the excitation and emission wavelengths to be transmitted to and received from the probe **30** with minimal interference. The probe **30** is preferably positioned within the retention chamber **19** proximate the bottom end **12** of the vessel **19**.

The vessel **10** may be formed from a wide variety of materials, such as various plastics (*e.g*., polypropylene or polyethylene terphthalate), glass, etc.

The radiation emitted by an excited probe **30** can be measured in terms of intensity and/or lifetime (rate of decay, phase shift or anisotropy), with measurement of lifetime generally preferred as a more accurate and reliable measurement technique.

Instruments (not shown) for interrogating probes **30** based on the quenching of photoluminescence by an analyte are well known and commercially available from various sources, including bioMérieux SA of France and Mocon, Inc. of Minneapolis, Minnesota.

### Method

In a first embodiment, the invention is a method of detecting the presence of *thermoduric microorganisms* in a product. The method includes the steps of (i) placing an aliquot **A** of the product into a vessel **10** equipped with a probe **30** sensitive to a target-analyte, (ii) pasteurizing the aliquot **A** within the vessel **10**, (iii) incubating the pasteurized aliquot **A** within the vessel **10** for an incubation period, typically at temperatures of between 30° to 55°C, and (iv) periodically interrogating the probe **30** during the incubation period.

The aliquot **A** within the vessel **10** may be pasteurized by any of the generally accepted pasteurization techniques, including Ultra High Temperature (UHT), High Temperature/Short Time (HTST), and Vat or Batch. For compliance purposes, it is preferred to use the same time and temperature values utilized with the currently employed LPC (*i.e.,* 63°C for 30 min). In order to avoid the problems associated with use of a water bath to achieve pasteurization temperatures, it is preferred to use a dry block heater to achieve and maintain stable pasteurization and incubation temperatures.

The interrogations measure changes in the probe **30** reflective of changes in concentration of a target-analyte within the aliquot **A**, thereby indicating the presence of viable *thermoduric microorganisms* in the aliquot **A**. Conversion algorithms used to convert the measured probe emissions to an oxygen concentration are well know to and readily developable by those with routine skill in the art.

If desired, the concentration of *thermoduric microorganisms* in the product prior to incubation can be determined by analyzing measured changes in the probe **30** in communication with the pasteurized aliquot A. Conversion algorithms for converting the measured emissions to a concentration of microorganisms in a sample are well know to and readily developable by those with routine skill in the art.

Nutrients can be added to the aliquot **A** prior to pasteurization for purposes of promoting growth of *thermoduric microorganisms* (or subsets thereof) in the aliquot **A**. Such nutrients, commonly referenced as culture media, are widely available from a number of sources. Persons of routine skill in the art would be able to identify and select suitable nutrients for incorporation into the aliquot **A**.

It is generally preferred to hermetically seal the aliquot **A** within the retention chamber **19** to facilitate handling (*e.g*., avoid spillage), prevent evaporation, and prevent both bacterial and target-analyte contamination of the aliquot **A**. The open top **11** of the retention chamber **19** can be sealed by any of the well know sealing techniques capable of withstanding the thermal treatment, including specifically but not exclusively a screw cap **20**, a heat or adhesive sealing foil (not shown), layer of mineral oil (not shown), etc.

The method is particularly suited for use in safety testing of liquid food products intended for human consumption, such as milk, juices and beverages.

In a second embodiment, the invention is a method for comparatively detecting the presence of *thermoduric microorganisms* and total microorganisms in a product. The method includes the steps of (i) obtaining a sample of the product, (ii) placing a first aliquot **A₁** of the sample into a first retention chamber **19₁** equipped with a first probe **30₁** sensitive to a target-analyte, (iii) placing a second aliquot **A₂** of the sample into a second retention chamber **19₂** equipped with a second probe **30₂** sensitive to a target-analyte, (d) pasteurizing the first aliquot **A₁** within the first retention chamber **19₁** but not the second aliquot **A₂,** (e) incubating the pasteurized first aliquot **A₁** within the first retention chamber **19₁** and the second aliquot **A₂** within the second retention chamber **19₂** for an incubation period, and (f) periodically interrogating both probes **30₁** and **30₂** during the incubation period, wherein the interrogations measure changes in the probe **30₁** or **30₂** reflective of changes in concentration of a target-analyte within the aliquot **A₁** or **A₂** respectively, with such changes in concentration indicative of the presence of viable *thermoduric microorganisms* in the first aliquot **A₁** and the presence of total viable microorganisms in the second aliquot **A₂**.

As with the first embodiment, the first aliquot **A₁** may be pasteurized by any of the generally accepted pasteurization techniques, including Ultra High Temperature (UHT), High Temperature/Short Time (HTST), and Vat or Batch. For compliance purposes, it is preferred to use the same time and temperature values utilized with the currently employed LPC (*i.e.,* 63°C for 30 min). In order to avoid the problems associated with use of a water bath to achieve pasteurization temperatures, it is preferred to use a dry block heater to achieve and maintain pasteurization temperatures.

The interrogations measure changes in the probe **30** reflective of changes in concentration of a target-analyte within the aliquot **A**, thereby indicating the presence of viable *thermoduric microorganisms* in the first aliquot **A₁** and the presence of total microorganisms in the second aliquot **A₂.** Again, conversion algorithms used to convert the measured probe emissions to an oxygen concentration are well know to and readily developable by those with routine skill in the art.

If desired, the concentration of *thermoduric microorganisms* in the product prior to incubation can be determined by analyzing measured changes in the first probe **30₁** to a concentration of *thermoduric microorganisms* in the pasteurized first aliquot **A₁,** and the concentration of total microorganisms in the product prior to incubation can be determined by analyzing measured changes in the second probe **30₂** to a concentration of total microorganisms in the pasteurized second aliquot **A₂**. Conversion algorithms used to convert the measured emissions to a concentration of microorganisms (thermoduric or total) in a sample are well know to and readily developable by those with routine skill in the art.

Nutrients can be added to both aliquots **A₁** and **A₂** prior to pasteurization / incubation for purposes of promoting growth of relevant microorganisms in the product.

As with the first embodiment, it is generally preferred to hermetically seal the aliquots **A₁** and **A₂** within the corresponding retention chamber **19₁** and **19₂** to prevent both bacterial and target-analyte contamination of the aliquot **A₁** and **A₂**.

As with the first embodiment, the method is particularly suited for use in safety testing of food products intended for human consumption, such as milk.

## Claims

1. A method of detecting the presence of thermoduric microorganisms in a product, comprising the steps of:
(a) placing an aliquot of the product into a vessel equipped with an optical probe sensitive to a thermoduric microorganism metabolite, wherein the thermoduric microorganism metabolite is oxygen, and wherein the optical probe is an oxygen sensitive photoluminescent dye,
(b) pasteurizing the aliquot within the vessel,
(c) incubating the pasteurized aliquot within the vessel for an incubation period, and
(d) periodically interrogating the probe during the incubation period, wherein the interrogations measure changes in the probe reflective of changes in concentration of a thermoduric microorganism metabolite within the aliquot, with such changes in concentration indicative of the presence of viable thermoduric microorganisms in the aliquot.

2. The method of claim 1 further comprising the steps of (i) converting measured changes in the probe to a concentration of thermoduric microorganisms in the pasteurized aliquot prior to incubation based upon a known conversion algorithm, and (ii) reporting the ascertained concentration of thermoduric microorganisms.

3. The method of claim 1 further comprising the step of hermetically sealing the aliquot within the retention chamber of the vessel prior to pasteurization.

4. The method of claim 1 wherein the product is a food product intended for animal or human consumption; orwherein the product is milk.

5. The method of claim 1 wherein the vessel is a vial, cuvette or multi-well plate; and/or wherein the vessel is a multi-well plate having more than 40 wells; and/or wherein the vessel defines a retention chamber having an elevated surface-to-volume ratio.

6. The method of claim 1 wherein pasteurization is effected at a defined temperature for a defined time period and thermal equilibration of the aliquot in the vessel is achieved within ½ or ¼ of the pasteurization time period.

7. The method of claim 1 wherein (i) the vessel defines a retention chamber having an open top end and a closed bottom end, and (ii) the probe is positioned within the retention chamber proximate the bottom end.

8. The method of claim 1 wherein the oxygen sensitive photoluminescent dye is an oxygen sensitive transition metal complex, wherein the oxygen sensitive transition metal complex is optionally selected from the group consisting of a ruthenium bipyridyl, a ruthenium diphenylphenanotroline, a platinum porphyrin, a palladium porphyrin, a phosphorescent complex of a tetrabenzoporphyrin, a chlorin, a porphyrin-ketone, an aza-porphyrin and a long-decay luminescent complex of iridium(III) or osmium(II).

9. The method of claim 1 wherein the aliquot within the vessel is pasteurized at 72°C for 20 seconds and incubated at 30° to 50°C for up to 24 hours; and/or wherein the aliquot within the vessel is pasteurized at 63°C for 30 minutes and incubated at 30° to 55°C for up to 24 hours; and/or wherein the aliquot within the vessel is incubated at 30°C

10. The method of claim 1 wherein pasteurization temperatures are achieved with a dry block heater; and/or wherein interrogations measure photoluminescence lifetime; and/or further comprising the step of adding nutrients effective for promoting growth of at least one *thermoduric microorganisms* to the aliquot prior to pasteurization.

11. A method for comparatively detecting the presence of *thermoduric microorganisms* and total microorganisms in a product, comprising the steps of:
(a) obtaining a sample of the product,
(b) placing a first aliquot of the sample into a first retention chamber equipped with a first probe sensitive to a thermoduric microorganism metabolite,
(c) placing a second aliquot of the sample into a second retention chamber equipped with a second probe sensitive to a target-analyte,
(d) pasteurizing the first aliquot within the first retention chamber but not the second aliquot,
(e) incubating the pasteurized first aliquot within the first retention chamber and the second aliquot within the second retention chamber for an incubation period, and
(f) periodically interrogating both probes during the incubation period, wherein the interrogations measure changes in the probe reflective of changes in concentration of a thermoduric microorganism metabolite within the first aliquot and changes in concentration of a target-analyte within the second aliquot, with such changes in concentration indicative of the presence of viable thermoduric microorganisms in the first aliquot and the presence of total viable microorganisms in the second aliquot, wherein the thermoduric microorganism metabolite and the target-analyte are both oxygen, and wherein the first and second probes are oxygen sensitive photoluminescent dye.

12. The method of claim 11 further comprising the steps of (i) converting measured changes in the first probe to a concentration of thermoduric microorganisms in the pasteurized first aliquot prior to incubation based upon a known conversion algorithm, (ii) converting measured changes in the second probe to a concentration of total microorganisms in the second aliquot prior to incubation based upon a known conversion algorithm, and (iii) reporting the ascertained concentration of thermoduric microorganisms and total microorganisms.

13. The method of claim 11 further comprising the step of hermetically sealing the first and second aliquots within their respective retention chambers prior to pasteurization and incubation and/or
wherein the product is a food product intended for human consumption; and/or
wherein the product is milk; and/or
wherein the first and second retention chambers are defined by separate and independent vials or cuvettes; and/or
wherein the first and second retention chambers are different wells in a muti-well plate; and/or wherein the multi-well plate has more than 40 wells; and/or
wherein the first and second retention chambers have an elevated surface-to-volume ratio.

14. The method of claim 20 wherein pasteurization is effected at a defined temperature for a defined time period and thermal equilibration of the aliquot in the vessel is achieved within ½ or 1/4 of the pasteurization time period; and/or
wherein (i) the first and second retention chambers have open top ends and closed bottom ends, and (ii) each probe is positioned within the respective retention chamber proximate the bottom end; and/or
wherein the oxygen sensitive photoluminescent dye is an oxygen sensitive transition metal complex, wherein the oxygen sensitive transition metal complex is optionally selected from the group consisting of a ruthenium bipyridyl, a ruthenium diphenylphenanotroline, a platinum porphyrin, a palladium porphyrin, a phosphorescent complex of a tetrabenzoporphyrin, a chlorin, a porphyrin-ketone, an aza-porphyrin and a long-decay luminescent complex of iridium(III) or osmium(II); and/or
wherein interrogations measure photoluminescence lifetime.

15. The method of claim 11 wherein the first aliquot is pasteurized at 72°C for 20 seconds and both the first and second aliquots are incubated at 30° to 50°C for up to 24 hours; and/or
wherein the first aliquot is pasteurized at 63°C for 30 minutes and both the first and second aliquots are incubated at 30° to 55°C for up to 24 hours; and/or
wherein the first aliquot is incubated at 30°C; and/or
wherein pasteurization temperatures are achieved with a dry block heater; and/or
further comprising the step of adding nutrients effective for promoting growth of microorganisms to the first and second aliquots prior to pasteurization and incubation.

16. The method of any preceding claims in which the oxygen sensitive photoluminescent dye is embedded within an oxygen-permeable polymer matrix.

## Patentansprüche

1. Verfahren zum Nachweis der Gegenwart von hitzebeständigen Mikroorganismen in einem Produkt, das die folgenden Schritte umfasst:
(a) Vorlegen eines Aliquots des Produkts in ein Gefäß, das mit einer optischen Sonde ausgestattet ist, die gegenüber einem Metaboliten von hitzebeständigen Mikroorganismen empfindlich ist, worin der Metabolit von hitzebeständigen Mikroorganismen Sauerstoff ist und worin die optische Sonde ein sauerstoffempfindlicher photolumineszierender Farbstoff ist,
(b) Pasteurisieren des Aliquots in dem Gefäß,
(c) Inkubieren des pasteurisierten Aliquots in dem Gefäß für eine Inkubationszeit und
(d) regelmäßiges Abfragen der Sonde während der Inkubationszeit, worin die Abfragen Veränderungen in der Sonde messen, die Veränderungen der Konzentration eines Metaboliten von hitzebeständigen Mikroorganismen in dem Aliquot widerspiegeln, wobei derartige Veränderungen der Konzentration die Gegenwart von lebensfähigen hitzebeständigen Mikroorganismen in dem Aliquot anzeigen.

2. Verfahren nach Anspruch 1, das weiter die folgenden Schritte umfasst: (i) Umwandeln von gemessenen Veränderungen in der Sonde in eine Konzentration an hitzebeständigen Mikroorganismen in dem pasteurisierten Aliquot vor der Inkubation, was auf einem bekannten Umwandlungsalgorithmus beruht, und (ii) Angeben der ermittelten Konzentration an hitzebeständigen Mikroorganismen.

3. Verfahren nach Anspruch 1, das weiter den Schritt des hermetischen Abdichtens des Aliquots innerhalb der Retentionskammer des Gefäßes vor der Pasteurisation umfasst.

4. Verfahren nach Anspruch 1, worin das Produkt ein Lebensmittelprodukt ist, das zum tierischen oder menschlichen Verzehr vorgesehen ist; oder worin das Produkt Milch ist.

5. Verfahren nach Anspruch 1, worin das Gefäß ein Fläschchen, eine Küvette oder Multi-Well-Platte ist; und/oder worin das Gefäß eine Multi-Well-Platte ist, die mehr als 40 Wells aufweist; und/oder worin das Gefäß eine Retentionskammer definiert, die ein erhöhtes Oberflächen-Volumen-Verhältnis aufweist.

6. Verfahren nach Anspruch 1, worin die Pasteurisation für eine definierte Zeitdauer bei einer definierten Temperatur durchgeführt wird und das thermische Gleichgewicht des Aliquots in dem Gefäß innerhalb der ½ oder des ¼ der Zeitdauer der Pasteurisation erreicht wird.

7. Verfahren nach Anspruch 1, worin (i) das Gefäß eine Retentionskammer definiert, die ein offenes oberes Ende und ein geschlossenes unteres Ende aufweist, und (ii) die Sonde innerhalb der Retentionskammer unmittelbar am unteren Ende positioniert wird.

8. Verfahren nach Anspruch 1, worin der sauerstoffempfindliche photolumineszierende Farbstoff ein sauerstoffempfindlicher Übergangsmetallkomplex ist, worin der sauerstoffempfindliche Übergangsmetallkomplex optional aus der Gruppe ausgewählt ist, die aus Folgenden besteht: einem Rutheniumbipyridyl, einem Rutheniumdiphenylphenanthrolin, einem Platinporphyrin, einem Palladiumporphyrin, einem phosphoreszierenden Komplex eines Tetrabenzoporphyrins, eines Chlorins, eines Porphyrin-Ketons, eines Azaporphyrins und einem lumineszierenden Komplex mit langer Abklingzeit von Iridium(III) oder Osmium(II).

9. Verfahren nach Anspruch 1, worin das Aliquot in dem Gefäß für 20 Sekunden bei 72°C pasteurisiert und für bis zu 24 Stunden bei 30°C bis 50°C inkubiert wird; und/oder worin das Aliquot in dem Gefäß für 30 Minuten bei 63°C pasteurisiert und für bis zu 24 Stunden bei 30°C bis 55°C inkubiert wird; und/oder worin das Aliquot in dem Gefäß bei 30°C inkubiert wird.

10. Verfahren nach Anspruch 1, worin die Temperaturen der Pasteurisation mit einem Trockenblock-Heizgerät erreicht werden; und/oder worin die Abfragen die Lebensdauer der Photolumineszenz messen; und/oder das weiter den Schritt der Zugabe von Nährstoffen, die für die Förderung des Wachstums von mindestens einem *hitzebeständigen Mikroorganismus* wirksam sind, zu dem Aliquot vor der Pasteurisation umfasst.

11. Verfahren für den vergleichenden Nachweis der Gegenwart von *hitzebeständigen Mikroorganismen* und gesamten Mikroorganismen in einem Produkt, das die folgenden Schritte umfasst:
(a) Erhalten einer Probe des Produkts,
(b) Vorlegen eines ersten Aliquots der Probe in eine erste Retentionskammer, die mit einer ersten Sonde ausgestattet ist, die gegenüber einem Metaboliten von hitzebeständigen Mikroorganismen empfindlich ist,
(c) Vorlegen eines zweiten Aliquots der Probe in eine zweite Retentionskammer, die mit einer zweiten Sonde ausgestattet ist, die gegenüber einem Zielanalyten empfindlich ist,
(d) Pasteurisieren des ersten Aliquots in der ersten Retentionskammer, doch nicht des zweiten Aliquots,
(e) Inkubieren des pasteurisierten ersten Aliquots in der ersten Retentionskammer und des zweiten Aliquots in der zweiten Retentionskammer für eine Inkubationszeit und
(f) regelmäßiges Abfragen beider Sonden während der Inkubationszeit, worin die Abfragen Veränderungen in der Sonde messen, die Veränderungen der Konzentration eines Metaboliten von hitzebeständigen Mikroorganismen in dem ersten Aliquot und Veränderungen der Konzentration eines Zielanalyten in dem zweiten Aliquot widerspiegeln, wobei derartige Veränderungen der Konzentration die Gegenwart von lebensfähigen hitzebeständigen Mikroorganismen in dem ersten Aliquot und die Gegenwart der gesamten lebensfähigen Mikroorganismen in dem zweiten Aliquot anzeigen, worin der Metabolit von hitzebständigen Mikroorganismen und der Zielanalyt Sauerstoff sind und worin die erste und zweite Sonde sauerstoffempfindlicher photolumineszierender Farbstoff sind.

12. Verfahren nach Anspruch 11, das weiter die folgenden Schritte umfasst: (i) Umwandeln von gemessenen Veränderungen in der ersten Sonde in eine Konzentration an hitzebeständigen Mikroorganismen in dem pasteurisierten ersten Aliquot vor der Inkubation, was auf einem bekannten Umwandlungsalgorithmus beruht, (ii) Umwandeln von gemessenen Veränderungen in der zweiten Sonde in eine Konzentration an gesamten Mikroorganismen in dem zweiten Aliquot vor der Inkubation, was auf einem bekannten Umwandlungsalgorithmus beruht, und (iii) Angeben der ermittelten Konzentration an hitzebständigen Mikroorganismen und gesamten Mikroorganismen.

13. Verfahren nach Anspruch 11, das weiter den Schritt des hermetischen Abdichtens des ersten und zweiten Aliquots in ihren jeweiligen Retentionskammern vor der Pasteurisation und Inkubation umfasst und/oder
worin das Produkt ein Lebensmittelprodukt ist, das zum menschlichen Verzehr vorgesehen ist; und/oder
worin das Produkt Milch ist; und/oder
worin die erste und zweite Retentionskammer durch separate und unabhängige Fläschchen oder Küvetten definiert sind; und/oder
worin die erste und zweite Retentionskammer verschiedene Wells in einer Multi-Well-Platte sind; und/oder worin die Multi-Well-Platte mehr als 40 Wells aufweist; und/oder
worin die erste und zweite Retentionskammer ein erhöhtes Oberflächen-Volumen-Verhältnis aufweisen.

14. Verfahren nach Anspruch 20 [*sic*], worin die Pasteurisation für eine definierte Zeitdauer bei einer definierten Temperatur durchgeführt wird und das thermische Gleichgewicht des Aliquots in dem Gefäß innerhalb der ½ oder des ¼ der Zeitdauer der Pasteurisation erreicht wird; und/oder
worin (i) die erste und zweite Retentionskammer offene obere Enden und geschlossene untere Enden aufweisen, und (ii) jede Sonde innerhalb der jeweiligen Retentionskammer unmittelbar am unteren Ende positioniert wird; und/oder
worin der sauerstoffempfindliche photolumineszierende Farbstoff ein sauerstoffempfindlicher Übergangsmetallkomplex ist, worin der sauerstoffempfindliche Übergangsmetallkomplex optional aus der Gruppe ausgewählt ist, die aus Folgenden besteht: einem Rutheniumbipyridyl, einem Rutheniumdiphenylphenanthrolin, einem Platinporphyrin, einem Palladiumporphyrin, einem phosphoreszierenden Komplex eines Tetrabenzoporphyrins, eines Chlorins, eines Porphyrin-Ketons, eines Azaporphyrins und einem lumineszierenden Komplex mit langer Abklingzeit von Iridium(III) oder Osmium(II); und/oder
worin die Abfragen die Lebensdauer der Photolumineszenz messen.

15. Verfahren nach Anspruch 11, worin das erste Aliquot für 20 Sekunden bei 72°C pasteurisiert wird und sowohl das erste als auch das zweite Aliquot für bis zu 24 Stunden bei 30°C bis 50°C inkubiert werden; und/oder
worin das erste Aliquot für 30 Minuten bei 63°C pasteurisiert wird und sowohl das erste als auch das zweite Aliquot für bis zu 24 Stunden bei 30°C bis 55°C inkubiert werden; und/oder
worin das erste Aliquot bei 30°C inkubiert wird; und/oder
worin die Temperaturen der Pasteurisation mit einem Trockenblock-Heizgerät erreicht werden; und/oder
das weiter den Schritt der Zugabe von Nährstoffen, die für die Förderung des Wachstums von Mikroorganismen wirksam sind, zu dem ersten und zweiten Aliquot vor der Pasteurisation und Inkubation umfasst.

16. Verfahren nach einem der vorstehenden Ansprüche, in dem der sauerstoffempfindliche photolumineszierende Farbstoff in einer sauerstoffdurchlässigen Polymermatrix eingebettet ist.

## Revendications

1. Méthode de détection de la présence de microorganismes thermoduriques dans un produit, comprenant les étapes consistant à
(a) placer une aliquote du produit dans un récipient équipé d'une sonde optique sensible à un métabolite de microorganisme thermodurique, où le métabolite de microorganisme thermodurique est l'oxygène, et où la sonde optique est un colorant photoluminescent sensible à l'oxygène,
(b) pasteuriser l'aliquote au sein du récipient,
(c) incuber l'aliquote pasteurisée au sein du récipient pendant une période d'incubation, et
(d) interroger périodiquement la sonde pendant la période d'incubation, où les interrogations mesurent les changements de la sonde reflétant les changements de concentration d'un métabolite de microorganisme thermodurique au sein de l'aliquote, de tels changements de concentration constituant une indication de la présence de microorganismes thermoduriques viables dans l'aliquote.

2. Méthode selon la revendication 1, comprenant en outre les étapes consistant à (i) convertir les changements mesurés de la sonde en une concentration en microorganismes thermoduriques dans l'aliquote pasteurisée préalablement à une incubation basée sur un algorithme de conversion connu, et (ii) rapporter la concentration établie en microorganismes thermoduriques.

3. Méthode selon la revendication 1, comprenant en outre l'étape consistant à sceller hermétiquement l'aliquote au sein de la chambre de rétention du récipient préalablement à la pasteurisation.

4. Méthode selon la revendication 1, dans laquelle le produit est un produit alimentaire prévu pour une consommation par l'animal ou l'homme ; ou dans laquelle le produit est du lait.

5. Méthode selon la revendication 1, dans laquelle le récipient est un flacon, une cuvette ou une plaque multi-puits ; et/ou dans laquelle le récipient est une plaque multi-puits ayant plus de 40 puits ; et/ou dans laquelle le récipient définit une chambre de rétention ayant un rapport élevé surface/volume.

6. Méthode selon la revendication 1, dans laquelle la pasteurisation est effectuée à une température définie pendant une période de temps définie et un équilibrage thermique de l'aliquote dans le récipient est effectué en moins de la moitié ou du quart de la période de temps de pasteurisation.

7. Méthode selon la revendication 1, dans laquelle (i) le récipient définit une chambre de rétention ayant une extrémité supérieure ouverte et une extrémité inférieure fermée, et (ii) la sonde est positionnée au sein de la chambre de rétention à proximité de l'extrémité inférieure.

8. Méthode selon la revendication 1, dans laquelle le colorant photoluminescent sensible à l'oxygène est un complexe de métal de transition sensible à l'oxygène, où le complexe de métal de transition sensible à l'oxygène est éventuellement choisi dans le groupe constitué par un bipyridyle de ruthénium, une diphénylphénanotroline de ruthénium, une porphyrine de platine, une porphyrine de palladium, un complexe phosphorescent d'une tétrabenzoporphyrine, une chlorine, une porphyrine-cétone, une aza-porphyrine et un complexe luminescent à dégradation lente d'iridium(III) ou d' osmium(II).

9. Méthode selon la revendication 1, dans laquelle l'aliquote au sein du récipient est pasteurisée à 72°C pendant 20 secondes et incubée à de 30° à 50°C pour une durée allant jusqu'à 24 heures ; et/ou dans laquelle l'aliquote au sein du récipient est pasteurisée à 63°C pendant 30 minutes et incubée à de 30° à 55°C pour une durée allant jusqu'à 24 heures ; et/ou dans laquelle l'aliquote au sein du récipient est incubée à 30°C.

10. Méthode selon la revendication 1, dans laquelle les températures de pasteurisation sont obtenues par un bloc chauffant à sec ; et/ou dans laquelle les interrogations mesurent la durée de vie de photoluminescence ; et/ou comprenant en outre l'étape consistant à ajouter des nutriments efficaces pour favoriser la croissance d'au moins un microorganisme thermodurique à l'aliquote préalablement à la pasteurisation.

11. Méthode destinée à détecter comparativement la présence de microorganismes thermoduriques et de microorganismes totaux dans un produit, comprenant les étapes consistant à :
(a) obtenir un échantillon du produit,
(b) placer une première aliquote de l'échantillon dans une première chambre de rétention équipée d'une première sonde sensible à un métabolite de microorganisme thermodurique,
(c) placer une deuxième aliquote de l'échantillon dans une deuxième chambre de rétention équipée d'une deuxième sonde sensible à un analyte cible,
(d) pasteuriser la première aliquote au sein de la première chambre de rétention, mais pas la deuxième aliquote,
(e) incuber la première aliquote pasteurisée au sein de la première chambre de rétention et la deuxième aliquote au sein de la deuxième chambre de rétention pendant une période d'incubation, et
(f) interroger périodiquement les deux sondes pendant la période d'incubation, où les interrogations mesurent les changements de la sonde reflétant les changements de concentration d'un métabolite de microorganisme thermodurique au sein de la première aliquote, et les changements de concentration d'un analyte cible au sein de la deuxième aliquote, de tels changements de concentration constituant une indication de la présence de microorganismes thermoduriques viables dans la première aliquote, et la présence de microorganismes viables totaux dans la deuxième aliquote, où le métabolite de microorganisme thermodurique et l'analyte cible sont tous deux l'oxygène, et où la première et la deuxième sonde sont un colorant photoluminescent sensible à l'oxygène.

12. Méthode selon la revendication 11, comprenant en outre les étapes consistant à (i) convertir les changements mesurés de la première sonde en une concentration en microorganismes thermoduriques dans la première aliquote pasteurisée préalablement à une incubation basée sur un algorithme de conversion connu, (ii) convertir les changements mesurés de la deuxième sonde en une concentration en microorganismes totaux dans la deuxième aliquote préalablement à une incubation basée sur un algorithme de conversion connu, et (iii) rapporter la concentration établie en microorganismes thermoduriques et en microorganismes totaux.

13. Méthode selon la revendication 11, comprenant en outre l'étape consistant à sceller hermétiquement la première et la deuxième aliquote au sein de leurs chambres de rétention respectives préalablement à la pasteurisation et l'incubation, et/ou
dans laquelle le produit est un produit alimentaire prévu pour une consommation par l'homme ; et/ou
dans laquelle le produit est du lait ; et/ou
dans laquelle la première et la deuxième chambre de rétention sont définies par des flacons ou des cuvettes séparés et indépendants ; et/ou
dans laquelle la première et la deuxième chambre de rétention sont des puits différents dans une plaque multi-puits ; et/ou dans laquelle la plaque multi-puits possède plus de 40 puits ; et/ou
dans laquelle la première et la deuxième chambre de rétention possèdent un rapport élevé surface/volume.

14. Méthode selon la revendication 20 *[sic]*, dans laquelle la pasteurisation est effectuée à une température définie pendant une période de temps définie et un équilibrage thermique de l'aliquote dans le récipient est effectué en moins de la moitié ou du quart de la période de temps de pasteurisation ; et/ou
dans laquelle (i) la première et la deuxième chambre de rétention possèdent des extrémités supérieures ouvertes et des extrémités inférieures fermées, et (ii) chaque sonde est positionnée au sein de la chambre de rétention respective à proximité de l'extrémité inférieure ; et/ou
dans laquelle le colorant photoluminescent sensible à l'oxygène est un complexe de métal de transition sensible à l'oxygène, où le complexe de métal de transition sensible à l'oxygène est éventuellement choisi dans le groupe constitué par un bipyridyle de ruthénium, une diphénylphénanotroline de ruthénium, une porphyrine de platine, une porphyrine de palladium, un complexe phosphorescent d'une tétrabenzoporphyrine, une chlorine, une porphyrine-cétone, une aza-porphyrine et un complexe luminescent à dégradation lente d'iridium(III) ou d' osmium(II) ; et/ou
dans laquelle les interrogations mesurent la durée de vie de photoluminescence.

15. Méthode selon la revendication 11, dans laquelle la première aliquote est pasteurisée à 72°C pendant 20 secondes et la première ainsi que la deuxième aliquote sont incubées à de 30° à 50°C pour une durée allant jusqu'à 24 heures ; et/ou
dans laquelle la première aliquote est pasteurisée à 63°C pendant 30 minutes et la première ainsi que la deuxième aliquote sont incubées à de 30° à 55°C pour une durée allant jusqu'à 24 heures ; et/ou
dans laquelle la première aliquote est incubée à 30°C ; et/ou
dans laquelle les températures de pasteurisation sont obtenues par un bloc chauffant à sec ; et/ou
comprenant en outre l'étape consistant à ajouter des nutriments efficaces pour favoriser la croissance de microorganismes à la première et la deuxième aliquote préalablement à la pasteurisation et l'incubation.

16. Méthode selon l'une quelconque des revendications précédentes, dans laquelle le colorant photoluminescent sensible à l'oxygène est inclus au sein d'une matrice polymère perméable à l'oxygène.
